# EUROPÄISCHE PATENTSCHRIFT

(11) **EP 1 594 948 B1**
(45) Veröffentlichungstag und Bekanntmachung des Hinweises auf die Patenterteilung: **02.12.2009**
(21) Anmeldenummer: 03775404.1
(22) Anmeldetag: 02.12.2003
(51) Int. Cl.: C12M 3/00, C12M 1/26, G01N 1/28

(54) **VERFAHREN UND VORRICHTUNGEN ZUR VERLETZUNGSFREIEN BEWEGUNG EINER SONDE DURCH BIOLOGISCHES ZELLMATERIAL**
METHOD AND DEVICES FOR NON-TRAUMATIC MOVEMENT OF A PROBE THROUGH BIOLOGICAL CELL MATERIAL
PROCÉDÉ ET DISPOSITIFS PERMETTANT DE DÉPLACER UNE SONDE SANS LÉSION À TRAVERS UNE MATIÈRE CELLULAIRE BIOLOGIQUE

(30) Priorität: 21.02.2003 DE 10307487
(43) Veröffentlichungstag der Anmeldung: 16.11.2005
(73) Patentinhaber: Fraunhofer-Gesellschaft zur Förderung der angewandten Forschung e.V., 80686 München (DE)
(72) Erfinder: FUHR, Günter, R., 13187 Berlin (DE); ZIMMERMANN, Heiko, 66386 St. Ingbert (DE)
(74) Vertreter: Hertz, Oliver
(86) Internationale Anmeldenummer: PCT/EP2003/013582
(87) Internationale Veröffentlichungsnummer: WO 2004/074426

(56) Entgegenhaltungen:
- EP-A- 0 539 888
- WO-A-03/047738
- CH-A- 652 933
- DE-B1- 2 501 270
- DE-C1- 19 714 987
- US-A- 5 877 008
- PATENT ABSTRACTS OF JAPAN Bd. 2000, Nr. 07, 29. September 2000 (2000-09-29) & JP 2000 098258 A (OLYMPUS OPTICAL CO LTD), 4. Juli 2000 (2000-07-04)
- PATENT ABSTRACTS OF JAPAN Bd. 016, Nr. 040 (C-0906), 31. Januar 1992 (1992-01-31) & JP 03 247268 A (SHIMADZU CORP), 5. November 1991 (1991-11-05)

## Beschreibung

Die Erfindung betrifft Verfahren zur Bewegung einer Sonde durch ein biologisches Zellmaterial, das aus tierischen oder menschlichen Zellen gebildet ist, Manipulations- und/oder Untersuchungseinrichtungen, die zur Durchführung derartiger Verfahren eingerichtet sind, Zellmanipulatoren, die mindestens eine derartige Manipulations- und/oder Untersuchungseinrichtung aufweisen, und Anwendungen der genannten Verfahren.

In der Medizin, Biotechnologie und Biochemie gibt es zahlreiche Verfahren, bei denen biologische Zellen untersucht oder bearbeitet oder zur Untersuchung oder Bearbeitung biologischen Materials verwendet werden. Beispielsweise werden zur medizinischen Zelltherapie einem tierischen oder menschlichen Probanden Zellen entnommen, außerhalb des Probandenkörpers behandelt, gesammelt, sortiert und/oder kultiviert, um anschließend bestimmte Zellen oder Zellgruppen in den Probanden zurückzuführen. Besondere Vorteile erwartet man von der medizinischen Zelltherapie mit Stammzellen, da diese die Fähigkeit zur Differenzierung zu nahezu allen Zelltypen des Körpers besitzen und daher Kandidaten für individuelle Zelltherapien und für die *in vitro* Regenerierung von Gewebe darstellen. Heute geht man davon aus, dass adulte oder embryonale Stammzellen unter geeigneten Bedingungen zu nahezu allen Zellleistungen des Körpers und damit auch zur Bildung oder Regeneration verschiedener Gewebe geeignet sind. Es besteht ein starkes Interesse an einer sicheren und reproduzierbaren Handhabung biologischer Zellen.

Wesentliche Aufgaben bei der Untersuchung oder Manipulation biologischer Zellen, insbesondere in Zusammenhang mit der medizinischen Zelltherapie und der Gewebebearbeitung (Tissue Engineering) bestehen darin, dass an vorbestimmten Orten bspw. im Gewebe oder in einem Zellverband mit einer Genauigkeit im µm-Bereich einzelne, vorher auswählbare Zellen oder Zellgruppen eingefügt oder entnommen oder definierte Messungen durchgeführt werden können. Diese Aufgaben müssen ohne Beeinträchtigung oder Schädigung der Zellen oder des Gewebes mit hoher Reproduzierbarkeit, Steuerbarkeit und Genauigkeit lösbar sein. Diese Anforderungen werden bisher nicht befriedigend erfüllt. Beispielsweise wurden bei Tierversuchen trotz gleichartig durchgeführter Verfahren, z. B. zur Injektion von Zellen in erkranktes Gewebe widersprüchliche Ergebnisse erzielt. Es wurde festgestellt, dass der positive Verlauf einer Geweberegeneration empfindlich von den Verfahrensbedingungen, insbesondere von der Art der Injektion, der Zahl der eingebrachten Zellen oder Substanzen und der verwendeten Injektionswerkzeuge abhängt. Bei zahlreichen, in der Praxis bekannt gewordenen Experimenten trat nicht die gewünschte Regeneration oder Neubildung eines Zell- oder Gewebetyps ein, sondern bspw. eine Induktion von Tumoren. Es wird davon ausgegangen, dass die Induktion von Tumoren als unkontrollierte Zellvermehrung von Stammzellen durch physikalische, chemische oder mechanische Fremdeinflüsse am Injektionsort befördert wird. Diese Einflüsse können mit den herkömmlichen Injektionstechniken nicht reproduzierbar eingestellt oder wenigstens erfasst werden.

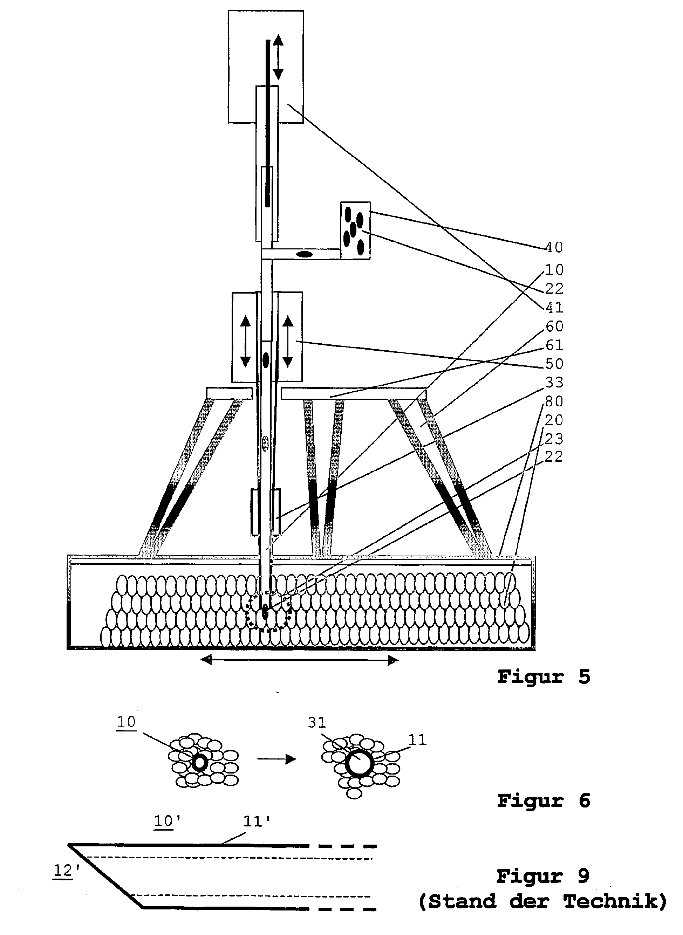

Bisher werden als Injektionswerkzeuge Kanülen oder Spritzennadeln verwendet. Beispielsweise zeigt Figur 9 die Spitze einer herkömmlichen Injektionskanüle 10' in vergrößerter Seitenansicht. Die Injektionskanüle 10' besitzt einen hohlen Kanülenkörper i1', dessen freies Ende 12' zur Bildung einer Spitze angeschrägt und ggf. geschliffen ist. Um einen möglichst geraden und reproduzierbaren Injektionskanal bspw. in einem Gewebe zu bilden, sind der Kanülenkörper 11' möglichst dünn und das Ende 12' spitz und scharf ausgeführt. Obwohl die herkömmliche Injektionskanüle 10' für Präzisionsanwendungen einen extrem geringen Durchmesser im Sub-mm-Bereich hat und das Ende 12' in eine Sub-µm-Spitze ausläuft, ist bei der herkömmlichen Anwendung der Kanüle eine verletzungsfreie Einführung in ein Gewebe grundsätzlich ausgeschlossen. Sobald die Spitze mit der bisher verwendeten hohen Einstichgeschwindigkeit in einem Gewebe auf Zellen trifft, werden diese mechanisch verletzt, zum Beispiel zerdrückt oder zerrissen.

Aus der Medizintechnik ist allgemein bekannt, subkutane Endoskopie durch Einschieben von Endoskopen oder Hilfsgeräten in subkutanes Gewebe durchzuführen. Die subkutane Endoskopie ist jedoch immer mit einer Verletzung von Gewebe und Blutgefäßen verbunden und daher für die biotechnologische Bearbeitung von Zellmaterialien ungeeignet.

Die genannten Probleme bei der Zelltherapie und die bisher zum Teil unbefriedigenden Ergebnisse beim Tissue Engineering stellen gegenwärtig die wichtigsten Beschränkungen und Verzögerungen vor einer breiten Anwendung dieser Verfahren in der Biotechnologie und der Medizin dar.

Die Aufgabe der Erfindung ist es, verbesserte Verfahren zur Bewegung einer Sonde durch ein Zellmaterial, das aus biologischen Zellen gebildet ist, bereitzustellen, mit denen den Problemen der herkömmlichen Injektionsverfahren begegnet wird und die grundsätzlich geeignet sind, die oben aufgeführten Anforderungen an die Zelltechniken zu erfüllen. Die Aufgabe der Erfindung ist es insbesondere, verbesserte Verfahren zur Einführung einer Sonde in Zellgewebe oder Zellhaufen bereitzustellen, mit denen in das Zellmaterial Zellen oder andere Substanzen einführbar, aus dem Zellmaterial Zellen oder Zellbestandteile entnehmbar und/oder im Zellmaterial Messungen durchführbar sind. Die Aufgabe der Erfindung ist es auch, verbesserte Manipulations- und/oder Untersuchungseinrichtungen zur Durchführung derartiger Verfahren und mit mindestens einer derartigen Einrichtung ausgestattete Injektionseinrichtungen bereitzustellen, mit denen die Nachteile herkömmlicher Injektionswerkzeuge überwunden werden. Eine weitere Aufgabe der Erfindung ist es, neue Anwendungen der Einführung von Sonden in Zellmaterial anzugeben.

Diese Aufgaben werden mit Verfahren, Sonden und Zellmanipulatoren mit den Merkmalen gemäß den Patentansprüchen 1, 14 oder 30 gelöst. Vorteilhafte Ausführungsformen und Anwendungen der Erfindung ergeben sich aus den abhängigen Ansprüchen.

Verfahrensbezogen basiert die Erfindung insbesondere auf dem Merkmal eine Sonde derartig durch ein Zellmaterial mit biologischen Zellen zu bewegen, dass die Sonde die Zellen verletzungsfrei verdrängt. Die Sonde wird so im Zellmaterial betätigt, dass die Zellen von der Oberfläche der Sonde auseinander geschoben oder voneinander getrennt werden, so dass Raum für die Sonde geschaffen wird, wobei die Zellen bei der Verschiebung oder Trennung in ihrem physikalischen und chemischen Zustand unverändert bleiben. Eine verletzungsfreie Verdrängung von Zellen ist insbesondere dann gegeben, wenn bei der Bewegung der Sonde die Zellen in direktem Kontakt mit dem Sondenkörper oder tiefer im Zellmaterial liegende Zellen zwar deformiert werden oder ihre räumliche Lage verändern, dabei jedoch keine chemischen Signale in Form von Botenstoffen oder Substanzabsonderungen abgeben.

Unter Zellmaterial wird hier allgemein eine Anhäufung von Zellen verstanden, die über Adhäsionskontakte (makromolekulare chemische Verbindungen, keine van der Waals-Bindungen) mit ihrer Umgebung in Verbindung stehen. Das Zellmaterial ist bspw. ein Verbund oder Zusammenschluss von Einzelzellen, ein Gewebe (Verband gleichartig differenzierter Zellen) oder ein Organ. Der nicht-flüssige Verbund von Einzelzellen kann zusätzliche synthetische Komponenten, zum Beispiel ein synthetisches Matrix- oder Trägermaterial enthalten. Vorteilhafterweise ergibt sich damit ein breiter Anwendungsbereich der Erfindung. Die Sonde ist allgemein ein Fremdkörper oder Objekt aus einem in Bezug auf das Zellmaterial abgrenzbaren Material, vorzugsweise mit einer festen Oberfläche. Die Sonde kann insbesondere eine Manipulations- und/oder Untersuchungseinrichtung wie eine Injektionskapillare oder wie elektrische Leitungen umfassen, die in das Zellmaterial (zum Beispiel Gehirn) eingeführt werden.

Die Erfindung basiert insbesondere auf den folgenden Überlegungen der Erfinder. Es wurde erstens erkannt, dass die bisher verschiedenartig ausfallenden Reaktionen bspw. injizierter Zellen in einem Gewebe oder einen Zellverband darin begründet liegen, dass durch die Einführung eines Injektionswerkzeugs Zellen im vorhandenen Zellmaterial verletzt oder zerstört und damit Wundeffekte hervorgerufen werden. Bei einer Zell- oder Gewebeverwundung werden chemische Signale (Aussendung molekularer Botenstoffe) oder zellulär getragene Prozesse, wie z. B. eine Fibroplasteneinwanderung, eine Fibronektinaussonderung oder dgl. erzeugt. Die Reaktion verletzter Zellen beeinflusst die Wirkung der injizierten Zellen oder Zusatzstoffe. Beispielsweise verhalten sich Stammzellen in der Umgebung einer Zellverwundung anders als Stammzellen in einem intakten Zellmaterial. Zweitens haben die Erfinder festgestellt, dass entgegen bisherigen Vorstellungen selbst adhäsiv gebundene Zellen verletzungsfrei räumlich verdrängt werden können. Dies ermöglicht die mechanische Einführung von Sonden in Zellmaterial. Die Zellen bleiben bei der Bewegung der Sonde durch das Zellmaterial unverletzt, wenn die Vortriebsgeschwindigkeit genügend klein ist, dass sich die Adhäsionskontakte zwischen den Zellen auf natürliche, d. h. die Zellen nicht beeinflussende oder zerstörende Weise lösen und in der sich verändernden Umgebung neu bilden lassen.

Durch die Bewegung der Sonde mittels verletzungsfreier Verdrängung von Zellen können die o. g. Anforderungen vollständig erfüllt werden. Weder das Zielgewebe noch die einzusetzenden individuellen Zellen oder Substanzen werden geschädigt oder beeinträchtigt. Der physikalische, chemische und mechanische Zustand der Zellen kann vollständig charakterisiert werden. Schädliche Kontakte zwischen Zellen und Oberflächen von Fremdkörpern werden vermieden, zelluläre Signale durch Oberflächenberührungen werden unterbunden. Durch die verletzungsfreie Bewegung erfolgt die Zellmanipulation ideal schonend. Die Sonde kann zielgenau an einen bestimmten Ort im Zellmaterial geführt werden. Besonders vorteilhaft ist auch, dass Beschränkungen an die Größe der Sonde, wie sie bei herkömmlichen Injektionskanülen bestanden, überwunden werden. Ein erfindungsgemäß durch das Zellmaterial bewegtes Werkzeug ermöglicht eine genaue und reproduzierbare Erfassung der Zahl und Art der in das Zellmaterial eingebrachten Zellen oder Zusatzstoffe.

Gemäß einer bevorzugten Ausführungsform der Erfindung wird die Sonde mit einer Vortriebsgeschwindigkeit bewegt, die kleiner oder gleich einer Bezugsgeschwindigkeit ist, die durch die physiologische Bindungsrate oder natürliche Zellbewegung biologischer Zellen bestimmt wird (Bindungsgeschwindigkeit der Zellen). Die natürliche Zellbewegung (englisch: cell locomotion) umfasst die Ortsänderung einer kompletten Zelle auf einer festen Oberfläche oder in Zellmaterial durch eine Umordnung von Adhäsionskontakten von Zellorganen (Membranorgane, zum Beispiel Membranausstülpungen), wie sie beispielsweise von M. Abercrombie et al. in der Publikation "The Locomotion Of Fibroblasts In Culture" ("Experimental Cell Research", Bd. 67, 1971, S. 359-367) und von L. P. Cramer in der Publikation "Organization and polarity of actin filament networks in cells: implications for the mechanism of myosin-based cell motility" ("Biochem. Soc. Symp." Bd. 65, 1999, S. 173-205) beschrieben wird.

Bei Einstellung der Vortriebsgeschwindigkeit kann die Sonde vorteilhafterweise verletzungsfrei durch Zellen in einem natürlich gegebenen Verbund bewegt werden. Die Vortriebsgeschwindigkeit wird an die permanent im Gewebe stattfindende Zellbewegung angepasst. Es ist bspw. bekannt, dass sich bestimmte Typen von Immunzellen (z. B. Makrophagen) durch Verdrängung vorhandener Zellen selbst durch dichtes Gewebe bewegen. Die Erfinder haben festgestellt, dass überraschenderweise diese Verdrängungsbewegung auch mit Sonden, die erheblich größer als Immunzellen sind und makroskopische Dimensionen im Sub-Millimeter- bis Zentimeterbereich besitzen, bei Einstellung der genannten Vortriebsgeschwindigkeit realisierbar ist. Während der Sondenbewegung werden laufend makromolekulare Bindungen (zum Beispiel membranständige Makromoleküle der Integrin- und Catherinfamilie) zwischen den Zellen getrennt und zum Beispiel mit der Sondenoberfläche neu geknüpft.

Die physiologische Bezugsgeschwindigkeit ist an sich bekannt (siehe z. B. G. Fuhr et al. in "Biol. Chem.", 1998, Bd. 379, S. 1161-1173) oder an tierischen oder humanen Zellen messbar. Die interessierende Bindungsrate ist bspw. durch Messung der Dynamik von Adhäsionsmustern einzelner Zellen auf künstlichen Oberflächen ableitbar.

Wenn die Sonde einer permanent wirkenden Vortriebskraft ausgesetzt wird, kann die Bewegung der Sonde mit der gewünschten Vortriebsgeschwindigkeit vorteilhafterweise selbst bei geringstem Kraftaufwand durchgeführt werden. Dies ermöglicht die Verwendung von Antriebseinrichtungen mit geringer Leistung. Wenn die Vortriebskraft durch eine mechanische Druckkraft gebildet wird, können sich Vorteile für die Übertragung der Vortriebskraft auf die Sonde ergeben. Wenn die Vortriebskraft durch Kräfte in elektrischen oder magnetischen Feldern gebildet wird, können sich Vorteile für den Aufbau einer Injektionseinrichtung ergeben, da die Vortriebskräfte über eine Fernwirkung ausgeübt werden können.

Gemäß einer besonderen Ausführungsform der Erfindung kann die Vortriebskraft durch interzelluläre Kräfte gebildet werden. Die Sonde kann unter der Wirkung der im Zellmaterial bestehenden Adhäsionsbindungen durch das Zellmaterial ohne einen äußeren Antrieb hindurch wandern. Wenn beispielsweise durch eine Oberflächenbehandlung der Sonde diese an einer Seite eine stärkere Neigung zur Bildung von Adhäsionsbindungen als an einer anderen Seite besitzt, kann die Sonde durch die Erzeugung der Adhäsionsbindungen vorwärtsgetrieben werden, was ggf. noch durch die Form der Sonde und/oder die anderen o. g. Vortriebskräfte unterstützt wird.

Gemäß einer bevorzugten Ausführungsform der Erfindung wird die Sonde in einer Richtung bewegt, die parallel zur Ausrichtung einer langgestreckten Form der Sonde verläuft. Dabei können sich Vorteile in Bezug auf die verletzungsfreie Verdrängung der Zellen ergeben. Die Verdrängung muss lediglich an der Vorderseite der Sonde erfolgen, die im Vergleich zur übrigen Oberfläche einen sehr kleinen Flächenausschnitt darstellt. Des Weiteren besitzt diese Ausführungsform den Vorteil, mit herkömmlichen Injektionstechniken unter Verwendung von Spritzen, Kanülen oder Kapillaren kompatibel zu sein. Die Sonde kann mit an sich verfügbaren Einrichtungen zur Manipulation von Zellen oder Zellsuspensionen kombiniert werden. Es wird insbesondere ermöglicht, dass mit der Sonde eine Substanz in das Zellmaterial zugeführt wird. Vorteilhafterweise können hierzu an sich bekannte Flüssigkeitsfördereinrichtungen, wie z. B. Pumpen oder dgl. verwendet werden.

Alternativ kann eine seitliche Bewegung der Sonde vorgesehen sein, bei der eine Bewegungsrichtung eingestellt wird, die von der z. B. langgestreckten Form der Sonde abweicht. Eine seitliche Bewegung der Sonde kann allgemein auch eine radiale Ausdehnung des Sondenkörpers im Zellmaterial umfassen (Aufweitungsbewegung).

Besondere Vorteile für die beabsichtigen Anwendungen in der Biotechnologie und Medizin können sich ergeben, wenn mit der Sonde mindestens eine Zelle in das Zellmaterial zugeführt wird, da die Zelle bei Injektion in das Zellmaterial dieses in einem physiologischen unverletzten Zustand vorfindet. Die mindestens eine Zelle wird in das Innere des unverletzten Zellmaterials eingebettet. Es können insbesondere Stammzellen in Gewebe eingepflanzt werden, um eine gewebespezifische Differenzierung der Stammzellen zu bewirken. Degenerationen oder Tumorbildungen können unterdrückt werden.

Vorteilhafterweise ermöglicht das erfindungsgemäße Verfahren auch die Injektion biologischer Zellen in einem gefrorenen Zustand. Nach einer Kryokonservierung können eine oder mehrere Zellen im gefrorenen Zustand in das Zellmaterial eingebettet und dort aufgetaut werden. Unmittelbar während des Auftauens werden die zellulären Prozesse unter den physiologischen Bedingungen im Zellmaterial gestartet.

Gemäß einer alternativen Ausführungsform der Erfindung wird mit der Sonde mindestens eine Zelle aus dem Zellmaterial entnommen. Die Sonde bildet ein Biopsiewerkzeug. Bei dieser Ausführungsform können sich Vorteile für die Gewinnung nichtmodifizierter, physiologischer Zellen ergeben.

Das erfindungsgemäße Prinzip der verletzungsfreien Verdrängung von Zellen ermöglicht die Verwendung von Sonden mit Dimensionen, die auch eine Integration von Sensoren ermöglichen. Gemäß einer Variante der Erfindung ist daher vorgesehen, dass mit der Sonde Eigenschaften des Zellmaterials, von injizierten Zellen oder von extrahierten Zellen erfasst werden.

Gemäß der Erfindung ist die Vortriebsgeschwindigkeit der Sonde in einem Geschwindigkeitsbereich von 0.1 µm/h bis 1 mm/h, vorzugsweise im Bereich von 1 µm/h bis zu 500 µm/h gewählt. In diesem Geschwindigkeitsbereich liegen die Bindungsraten des Aufbaus und des Abbaus von makromolekularen Bindungen, die typischerweise durch membranständige Makromoleküle der Integrin- und Catherinfamilie vermittelt werden. Die bevorzugten Geschwindigkeitsbereiche entsprechen den Geschwindigkeiten der Zellbewegung von insbesondere Fibroplasten, Makrophagen, Lymphozyten, Chondrozyten oder Tumorzellen. Vorteilhafterweise kann bei Einstellung einer derart geringen Vortriebsgeschwindigkeit die Position der Sonde mit einer hohen Genauigkeit von bis zu +/-1 µm eingestellt werden. Die Vortriebsgeschwindigkeiten in den genannten Bereichen entsprechen den aktiven endogenen Bewegungsgeschwindigkeiten von Zellen in und auf Gewebe. Die Bewegung der Sonde bewirkt damit einen permanenten An- und Umbau der Zellen in der unmittelbaren Umgebung der Sondenoberfläche, wobei durch die permanent wirkende Vortriebskraft eine Verdrängung der Zellen gefördert wird.

Vorteilhafterweise können je nach Anwendungsfall verschiedene Bewegungstypen der Sonde, insbesondere Bewegungen mit einem Netto-Vorschub oder einem Netto-Rückzug, diskontinuierliche oder in Teilabschnitte zerlegte Bewegungen, oszillatorische, gleichförmige oder beschleunigte Bewegungen realisiert werden.

Besondere Vorteile können sich ergeben, wenn das erfindungsgemäße Verfahren an Zellmaterial ausgeführt wird, das sich außerhalb eines tierischen oder menschlichen Organismus befindet. Das Zellmaterial kann unter geeigneten Kultivierungsbedingungen auf einem festen Träger angeordnet werden, der die Gegenkraft zur Ausübung der Vortriebskraft aufbringt. Das Zellmaterial und die Sonde können mit hoher Präzision positioniert werden.

Ein weiterer Gegenstand der Erfindung ist eine Sonde , die dazu eingerichtet ist, zumindest teilweise in das Zellmaterial eingeführt zu werden. Die Sonde besitzt einen Sondenkörper, der durch das Zellmaterial beweglich ist und an mindestens einer Vorderseite, die bei einer Bewegung der Sonde durch das Zellmaterial ein relativ zur Bewegungsrichtung vorderes Ende, Frontende oder vorderes Verdrängungs- oder Trennteil bildet, insbesondere eine abgerundete oder abgeplattete Oberfläche aufweist. Die Vorderseite besitzt eine Kontur, die frei von Spitzen, Stufen, Kanten, Schneiden und dgl. ist. Die Vorderseite besitzt eine mathematisch stetige Krümmungskontur, die als Ausschnitt einer Oberfläche einer Kugel, eines Ellipsoiden, eines Toroiden oder eine Überlagerung aus diesen beschrieben werden kann. Die Bereitstellung der abgerundeten oder abgeplatteten Oberfläche an mindestens einer Vorderseite besitzt den Vorteil, dass beim Einführen der Sonde entsprechend dem erfindungsgemäßen Verfahren im Zellmaterial Verletzungen vermieden werden, da die Vortriebskraft gleichmäßig über die gesamte Abrundung der Vorderseite wirkt. Lokale Druckerhöhungen auf einzelne Zellen, die bspw. durch Spitzen oder Stufen verursacht werden, können mit dem erfindungsgemäßen Werkzeug ausgeschlossen werden. Damit unterscheidet sich die Sonde grundsätzlich von herkömmlichen Injektionswerkzeugen, wie z. B. Spritzennadeln, bei denen die Verletzung notwendig durch die Führung eines Schnittes durch das Zellmaterial gegeben ist.

Gemäß einer bevorzugten Ausführungsform der Erfindung besitzt die Sondenvorderseite in einzelnen Bereichen der abgerundeten Oberfläche einen lokalen Krümmungsradius, der größer als 10 µm ist. Damit ist die abgerundete oder abgeplattete Oberfläche größer als die Zellarten in den typischerweise behandelten Zellmaterialien, insbesondere Zellarten in Gewebe. Damit wird die Verletzungswahrscheinlichkeit bei der Bewegung der Sonde vermindert. Besondere Vorteile können sich mit einem lokalen Krümmungsradius ergeben, der größer als 20 µm, vorzugsweise größer als 0.1 mm ist. Für die Anwendung bei Zellkulturen ist der Krümmungsradius kleiner als 5 mm, vorzugsweise kleiner als 2 mm. Alternativ kann er sehr groß sein, so dass die Vorderseite im wesentlichen abgeplattet ist.

Gemäß der Erfindung trägt die abgerundete Oberfläche eine Beschichtung mit einem Material (Bindungsmaterial) gebildet, das ein adhärentes Anhaften von Zellen auf der Oberfläche fördert. Das Bindungsmaterial bildet den Sondenkörper, mindestens die Vorderseite des Sondenkörpers oder eine Beschichtung mindestens auf der Vorderseite des Sondenkörpers. Es besteht bspw. aus Fibronektin oder Kollagen. Diese Ausführungsform der Erfindung kann Vorteile in Bezug auf eine Erhöhung der Bindungsgeschwindigkeit bei der verdrängenden Bewegung der Sonde durch das Zellmaterial besitzen. Das Bindungsmaterial kann alternativ durch eine Aufrauhung mit charakteristischen Strukturgrößen im Sub-µm-Bereich besitzen, so dass die Anbindung des Zellmaterials an die Sonde gefördert wird. Ebenso wie die Abrundung mindestens einer Oberfläche stellt auch die Verwendung eines adhäsionsfördernden Materials einen wesentlichen und grundsätzlichen Unterschied des erfindungsgemäßen Werkzeugs gegenüber herkömmlichen Injektionsnadeln dar.

Gemäß einer vorteilhaften Variante der Erfindung ist in den Sondenkörper mindestens ein Funktionsteil integriert. Das Funktionsteil stellt allgemein eine strukturelle Komponente des Sondekörpers dar, die für eine bestimmte technische Funktion der Sonde eingerichtet ist. Vorteilhafterweise ist das Funktionsteil in den Sondenkörper integriert, so dass die verletzungsfreie Verdrängungsbewegung nicht gestört wird.

Das Funktionsteil umfasst gemäß einer bevorzugten Ausführungsform der Erfindung einen Hohlraum der zur Aufnahme und/oder Leitung von Zellen oder Zusatzstoffen in oder aus dem Zellmaterial eingerichtet ist. Der im Sondenkörper gebildete Hohlraum besitzt einerseits mindestens eine Öffnung, die in einer Oberfläche des Werkzeugskörpers gebildet ist. Andererseits kann ein Anschluss des Hohlraums an ein externes Probenreservoir vorgesehen sein.

Des Weiteren kann das Funktionsteil mindestens einen Sensor im Sondenkörper umfassen, der zur Erfassung von chemischen oder physikalischen Eigenschaften von Zellen oder Substanzen im Zellmaterial oder im Inneren des Sondenkörpers eingerichtet ist. Wenn der mindestens eine Sensor im Hohlraum des Sondenkörpers angeordnet ist, können sich Vorteile für die Überwachung einer Injektion oder einer Biopsie ergeben.

Das Funktionsteil kann ferner mindestens einen elektrischen Leiter umfassen, der bis in die abgerundete Vorderseite der Sonde verläuft. Der Leiter kann zum Beispiel in die Sonde eingeschmolzen sein und eine Messelektrode bilden. Es kann auch mindestens ein Lichtleiter zum Beispiel für spektroskopische Messungen im Zellmaterial vorgesehen sein.

Gemäß einer weiteren bevorzugten Ausführungsform der Erfindung ist vorgesehen, dass der Sondenkörper eine gerade, langgestreckte Form entlang einer geraden Achse (Kapillare, Rohr, Hohlnadel) mit einem ersten Ende, das die abgerundete Vorderseite ist, und einem zweiten Ende gebildet wird, das mit einer Reservoireinrichtung verbunden ist. Das erste Ende kann eine Abrundung der Kapillarwand (ringförmige Abrundung) oder das Ende des Sondenkörpers (kugelförmige Abrundung) umfassen. Bei diesen Gestaltungen ist der Sondenkörper vorzugsweise eine Spritzennadel mit einem freien Ende, an dem die Wand der Spritzennadel abgerundet ist. Diese Ausführungsform der Erfindung kann wegen ihrer Kompatibilität mit herkömmlichen Injektionswerkzeugen Vorteile besitzen.

Wenn die erfindungsgemäße Sonde an ihrem freien Ende ein Aufnahmewerkzeug aufweist, können sich weitere Vorteile bei der Aufnahme oder Ablage von Einzelzellen an Zellmaterial (z. B. Zellkulturen) ergeben. Das Aufnahmewerkzeug besitzt eine Aufnahmefläche, die als Substrat oder Träger für mindestens eine Zelle dient und eine konkave oder konvexe Form besitzt. Eine konkave Aufnahmefläche kann Vorteile in Bezug auf die Abgrenzung des Aufnahmewerkzeugs von der Umgebung, wie z. B. von übrigen Sondenteilen oder von Zellmaterial besitzen. Der Vorteil der konvexen Aufnahmefläche besteht in deren optimaler Anpassung an die erfindungsgemäße verdrängende Bewegung der Sonde durch das Zellmaterial.

Wenn gemäß einer Variante der Erfindung das Aufnahmewerkzeug in einer kapillarförmigen Sonde oder in einer Sonde mit einer rinnenförmigen Ausnehmung angeordnet ist, wird vorteilhafterweise die Bereitstellung einer Suspensions- oder Kultivierungsflüssigkeit in der Umgebung des Aufnahmewerkzeugs und der auf diesem befindlichen mindestens einen Zelle erleichtert. Weitere Vorteile für die Ablage von Zellen aus der Kapillare oder der rinnenförmigen Ausnehmung ergeben sich, wenn das Aufnahmewerkzeug in der Sonde verschiebbar angeordnet ist.

Alternativ kann der Sondenkörper durch einen Formkörper gebildet werden, dessen gesamte Oberfläche abgerundet wie die o. g. Vorderseite ist. Vorteilhafterweise kann der Sondenkörper somit vollständig vom Zellmaterial umhüllt ohne mechanische Verbindung mit zusätzlichen äußeren Einrichtungen durch das Zellmaterial bewegt werden. Der Sondenkörper kann bspw. komplett kugelförmig gebildet sein. Gemäß einer Abwandlung dieser Ausführungsform kann vorgesehen sein, dass der Sondenkörper durch ein kugelförmiges Aufnahmewerkzeug mit einer stabförmigen Halterung gebildet wird, mit der vorteilhafterweise eine Vortriebskraft von einer Antriebseinrichtung auf das Aufnahmewerkzeug übertragen werden kann.

Bei der Anwendung der Erfindung zur Aufnahme oder Ablage von Zellen an einem Zellträger, z. B. mit einer Zellkultur kann es von Vorteil sein, wenn die Sonde mit mindestens einem Abstandshalter ausgestattet ist, mit dem der Sondenkörper auf dem Zellträger aufsetzbar ist. Dadurch kann vorteilhafterweise die Genauigkeit und Stabilität der Positionierung der Sonde auf dem Zellträger erhöht werden.

Wenn der Sondenkörper mit mindestens einem Kraftelement ausgestattet wird, können sich Vorteile für die gezielte und gerichtete Einwirkung einer äußeren Vortriebskraft ergeben. Wenn das Kraftelement eine mechanische Halterung ist, ergeben sich Vorteile in Bezug auf die Zuverlässigkeit und Präzision der Kraftübertragung. Wenn das Kraftelement ein Magnetelement umfasst, wird vorteilhafterweise eine berührungsfreie Manipulation der Sonde im Zellmaterial ermöglicht.

Ein weiterer Gegenstand der Erfindung ist ein Zellmanipulator (insbesondere ein Arbeitsgerät wie zum Beispiel eine Injektions-, Biopsie- und/oder Untersuchungseinrichtung für die Bearbeitung von Zellmaterial, wobei der Zellmanipulator mindestens eine erfindungsgemäße Sonde und mindestens eine Antriebseinrichtung zur Bewegung der mindestens einen Sonde umfasst. Der Zellmanipulator besitzt den besonderen Vorteil, dass die Sonde mit der Antriebseinrichtung mit hoher Genauigkeit und Reproduzierbarkeit im Zellmaterial manipuliert werden kann. Wenn die Antriebseinrichtung einen piezoelektrischen Antrieb umfasst, ergeben sich Vorteile für die Steuerbarkeit der Sondenbewegung. Alternativ kann die Antriebseinrichtung durch einen Magnetantrieb gebildet werden, wodurch sich Vorteile in Bezug auf eine berührungslose Übertragung der Vortriebskraft ergeben. Des Weiteren kann die Antriebseinrichtung einen Federantrieb umfassen, so dass sich Vorteile in Bezug auf einen besonders einfachen Aufbau der Manipulationseinrichtung ergeben.

Gemäß bevorzugten Ausführungsformen der Erfindung ist der Zellmanipulator mit einer Positioniereinrichtung zur Steuerung der Antriebseinrichtung, einer Detektoreinrichtung zur Erfassung der Position des mindestens einen Werkzeugs und/oder einer Trägereinrichtung zur Aufnahme von Zellmaterial ausgestattet.

Bevorzugte Anwendungen der Erfindung sind die in vitro Zellkultur, das Tissue Engineering in der Biotechnologie, die Schaffung von Gewebemodellen für die Pharmakologie und die medizinische Therapie.

Weitere Einzelheiten und Vorteile der Erfindung werden im Folgenden unter Bezug auf die beigefügten Zeichnungen beschrieben. Es zeigen:
Figur 1: den Ablauf einer Zellinjektion entsprechend einer Ausführungsform des erfindungsgemäßen Verfahrens,
Figur 2: verschiedene Ausführungsformen erfindungsgemäßer Sonden,
Figur 3: eine abgewandelte Ausführungsform einer erfindungsgemäßen Sonde,
Figur 4: eine schematische Illustration eines erfindungsgemäßen Zellmanipulators,
Figur 5: eine Illustration von weiteren Einzelheiten einer Ausführungsform eines erfindungsgemäßen Zellmanipulators,
Figur 6: eine Illustration einer radialen Aufweitungsbewegung einer Sonde, und
Figur 7: eine weitere abgewandelte Ausführungsform einer erfindungsgemäßen Sonde,
Figur 8: den Ablauf einer Zellformung entsprechend einer Ausführungsform des erfindungsgemäßen Verfahrens,
Figur 9: die Spitze einer herkömmlichen Injektionsnadel.

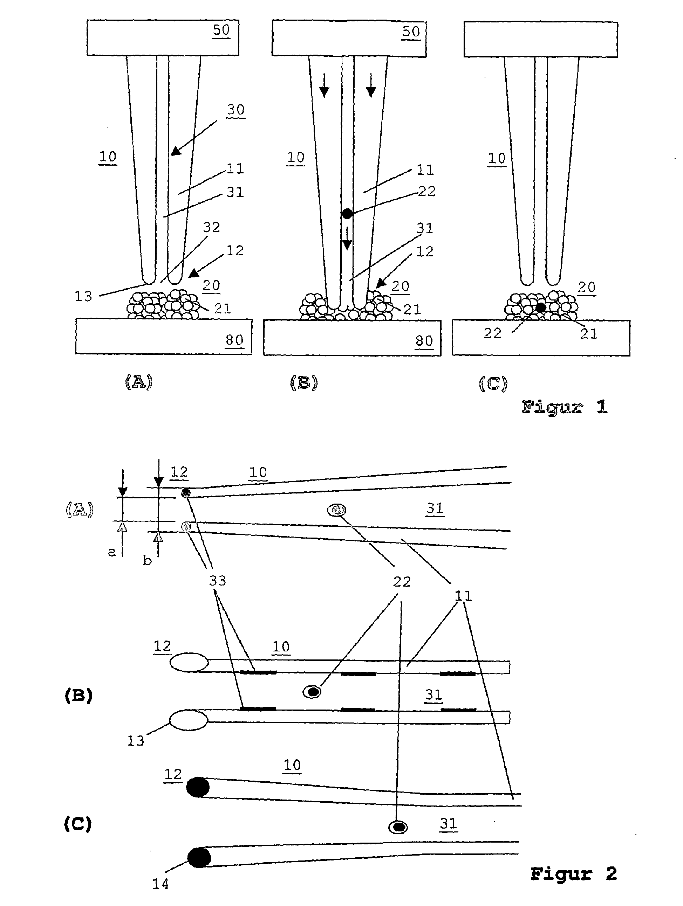

Die erfindungsgemäße Bewegung einer Sonde durch ein Zellmaterial ist in Figur 1 am Beispiel der Injektion einer Zelle in eine Gewebeprobe gezeigt. Im linken Teil A von Figur 1 ist eine erfindungsgemäße Sonde 10 in schematischer Schnittansicht illustriert. Die Sonde 10 umfasst einen Sondenkörper 11, der durch eine Hohlnadel oder Pipettenspitze gebildet wird. Das freie Ende 12 des Sondenkörpers 11 stellt die Vorderseite 13 mit der erfindungsgemäß abgerundeten Oberfläche dar. Im Innern des Sondenkörpers 11 verläuft als Funktionsteil 30 ein Hohlkanal 31 mit einer Öffnung 32 am Ende 12. Das entgegengesetzte Ende des Sondenkörpers 11 ist mit einer piezoelektrischen Antriebseinrichtung 50 verbunden.

In einer Ausgangsposition (A) befindet sich die Sonde 10 mit Abstand von einem Zellmaterial 20, das auf einem Träger 80 angeordnet ist. Das Zellmaterial 20 ist bspw. ein Zellhaufen oder Sphäroid aus 50 bis 500 aneinander grenzenden Zellen 21, die über Adhäsionskontakte miteinander verbunden sind. In dieses Zellmaterial 20 sollen mit der nachfolgend beschriebenen Prozedur eine Stammzelle injiziert werden, um diese im Zellmaterial zu einer zellspezifischen Differenzierung zu veranlassen.

Gemäß dem mittleren Teilbild B von Figur 1 wird die Sonde 10 in das Zellmaterial 20 geschoben, wobei die Zellen 21 des Zellmaterials verdrängt werden. Sobald das Ende 12 auf dem Zellmaterial 20 aufsitzt, bilden sich Adhäsionskontakte mit den Zellen 12. Unter der Wirkung der permanent wirkenden Vortriebskraft der Antriebseinrichtung 50 werden die Adhäsionskontakte laufend umgeordnet, so dass zunächst das Ende und mit fortschreitendem Vortrieb auch der sich anschließende Teil der Sondenkörpers 11 von den Zellen 21 umgeben werden. Während der Bewegung der Sonde durch das Zellmaterial erfolgt ein laufendes Umwachsen der Werkzeugoberfläche mit den Zellen 21.

Wenn das Ende 12 einen vorbestimmten Abstand (z. B. halbe Dicke des Zellhaufens) vom Träger 80 besitzt, wird die Vortriebsbewegung gestoppt. Von einer (nicht dargestellten) Reservoireinrichtung wird eine Stammzelle 22 durch den Hohlkanal 31 in das Zellmaterial 20 bewegt. Diese Bewegung erfolgt bspw. durch Einspülen mit einer Suspensionsflüssigkeit.

Nach der Injektion der Stammzellen in das Zellmaterial wird die Sonde 10 zurückgezogen. Die Rückzugsbewegung erfolgt ebenfalls mit einer derart geringen Geschwindigkeit, dass sich die Zellen 21 verletzungsfrei umordnen können, bis der verdrängte Raum um die injizierte Stammzelle 22 wieder gefüllt ist (Teilbild C von Figur 1).

Alternativ zur Gestaltung gemäß Figur 1 kann vorgesehen sein, dass die Antriebseinrichtung 50 am Träger 80 angebracht ist und die Relativbewegung zwischen dem Zellmaterial 20 und der Sonde 10 trägerseitig erzeugt wird, während die Sonde 10 fest positioniert ist.

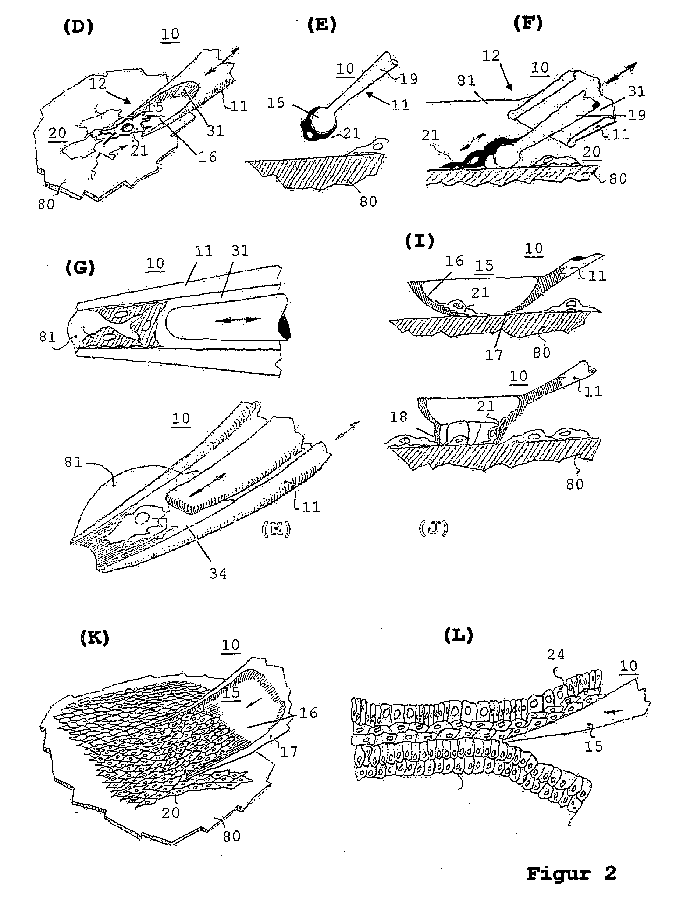

In Figur 2 sind weitere Einzelheiten des freien Endes 12 bei verschiedenen Ausführungsformen erfindungsgemäßer Sonden 10 illustriert. Teilbild (A) zeigt einen sich konisch verjüngenden Sondenkörper 11. Durch diese Form wird vorteilhafterweise die Verdrängung von Zellen im Zellmaterial begünstigt. Die Vorderseiten 13 sind entsprechend den oben erläuterten Prinzipien abgerundet. Der Sondenkörper 11 besteht bspw. aus Glas, einem inerten Metall (z. B. Platin) oder einem inerten Kunststoffmaterial (z. B. Polyimid). Die charakteristischen Dimensionen a (Innendurchmesser des Hohlkanals 31 an der Öffnung 32) und b (Außendurchmesser des Sondenkörpers 11 am Ende 12) sind typischerweise in den Bereichen a = 10 µm bis 100 µm und b = 10.5 µm bis 200 µm gewählt. Es können kleinere Maße im Bereich a = 0.1 µm bis 10 µm für die Injektion von besonders kleinen Zellen oder von synthetischen Partikeln oder größere Maße von z. B. a = 100 µm bis 5 mm für die Injektion z. B. von embryonalen Stammzellen (ausgenommen menschliche embryonale stammzellen) gewählt werden, wobei das Maß b entsprechend der Wandstärke des Sondenkörpers 11 entsprechend größer gewählt ist.

Die Sonde 10 kann mit einem Sensor 33 ausgestattet sein. Gemäß Teilbild (A) ist ein Impedanzsensor 33 am Ende 12 vorgesehen. Der Impedanzsensor 33 umfasst zwei halbkreisförmige Elektroden (Impedanzelektroden), die auf der Oberfläche der Vorderseite 13 bspw. durch Aufdampfen angebracht sind. Alternativ können die Impedanzelektroden auf der Außenseite des Sondenkörpers 11 an dessen Ende 12 angebracht sein. Über elektrische Verbindungsleitungen (nicht gezeigt) entlang dem Sondenkörper 11 sind die Elektroden des Impedanzsensors 33 mit einer Steuereinrichtung verbunden. Beim Durchströmen einer Zelle 22 durch den Hohlkanal 31 wird durch die Verstimmung der Impedanz zwischen den Impedanzelektroden ein Impedanzsignal generiert, das in an sich bekannter Weise Auskunft über die Zahl, Größe und die elektrischen Eigenschaften der vorbeitretenden Zelle gibt.

Gemäß Teilbild (B) können Impedanzsensoren 33 alternativ oder zusätzlich auf der Innenseite des Hohlkanals 31 vorgesehen sein. Des Weiteren zeigt Teilbild (B) am Ende 12 des mit einem konstanten Durchmesser gebildeten Sondenkörpers 11 eine sich auswölbende Vorderseite 13.

Gemäß Teilbild (C) kann der Sondenkörper 11 sich zum Ende 12 hin konisch erweitern. Teilbild (C) von Figur 2 illustriert schematisch eine adhäsionsfördernde Beschichtung 14 auf dem Ende 12 des Sondenkörpers 11. Diese Verbindungsbeschichtung ist vorzugsweise nur auf der abgerundeten, verdrängenden Vorderseite der Sonde 10 vorgesehen, während die Oberfläche des übrigen Sondenkörpers 11 vorzugsweise adhäsionsmindernd beschichtet ist, um ein Gleiten zwischen den verdrängten Zellen zu fördern. Die Verbindungsbeschichtung besteht zum Beispiel aus Fibronektin. Zur Adhäsionsminderung kann eine Silanisierung oder eine Belegung mit biologischen Makromolekülen (zum Beispiel PolyHema) vorgesehen sein. Auch die innere Oberfläche des Hohlkanals kann in dieser Weise adhäsionsmindernd beschichtet sein.

Die Querschnittsform der kapillarförmigen Sondenkörper ist vorzugsweise rund. Alternativ kann eine abgeflachte Ellipsenform vorgesehen sein, die eine Vorzugsrichtung zur seitlichen Bewegung in einer Bewegungsrichtung senkrecht zur Längsachse des Sondenkörpers 11 ermöglicht.

Bei der in Teilbild (D) gezeigten Ausführungsform der Erfindung ist der kapillarförmige Sondenkörper an seinem freien Ende 12 schräg angeschnitten, so dass ein schaufelförmiges Aufnahmewerkzeug 15 gebildet wird. Die Innenwand des Hohlkanals 31 der Kapillare 11 bildet am freien Ende 12 eine Aufnahmefläche 16, die seitlich durch hochstehende Ränder 17 begrenzt wird. Zum bestimmungsgemäßen Gebrauch wird die Sonde 10 auf einen Träger 80 (z. B. Boden eines Kulturträgers) oder ein auf diesem befindliches Zellmaterial 20 aufgesetzt. Die Sonde 10 wird im Zellmaterial 20 vorgeschoben (siehe Doppelpfeil) bis eine gewünschte Zelle 21 auf das Aufnahmewerkzeug 15 transferiert worden ist. Der Zelltransfer kann durch die natürliche Eigenbewegung der Zelle 21 gefördert werden. Sobald die Zelle 21 von der Sonde 10 aufgenommen wurde, kann diese vom Träger 80 entfernt und zu einem Zielsubstrat bewegt werden.

Gemäß Teilbild E umfasst der Sondenkörper 11 der erfindungsgemäßen Sonde 10 ein Aufnahmewerkzeug 15, das am Ende eines Sondenschaftes angeordnet ist. Das Aufnahmewerkzeug 15 besitzt eine Kugelform, deren Oberfläche eine Aufnahmefläche für eine von einem Träger 80 zu transferierende Zelle 21 bildet. Das Aufnahmewerkzeug 15 besitzt einen Durchmesser von z. B. 15 µm.

Das kugelförmige Aufnahmewerkzeug 15 kann gemäß Teilbild F der Figur 2 mit einem kapillarförmigen Sondenkörper 11 kombiniert werden. Das Aufnahmewerkzeug 15 ist mit dem Sondenschaft 19 im Hohlkanal 31 entsprechend der Längsachse der Kapillare 11 verschiebbar. Zur Aufnahme einer Zelle 21 wird die Sonde 10 in eine Kultivierungsflüssigkeit 81 über dem Träger eingetaucht und an das auf diesem befindliche Zellmaterial 20 angenähert. Das Aufnahmewerkzeug 15 wird mit einer Antriebseinrichtung (siehe unten) vorgeschoben, wobei Zellmaterial 20 ggf. verletzungsfrei verdrängt wird. Durch die Vorschubbewegung und ggf. durch die natürliche Zellbewegung unterstützt, kann die Zelle 21 auf das Aufnahmewerkzeug 15 transferiert werden. Teilbild F illustriert auch die abgerundete Form aller Teile des freien Endes 12 der Sonde 10, so dass diese komplett entsprechend dem erfindungsgemäßen Verfahren verletzungsfrei durch Zellmaterial bewegt werden kann.

Weitere Varianten eines relativ zum übrigen Sondenkörper 11 verschiebbaren Aufnahmewerkzeugs 15 sind in den Teilbildern G und H der Figur 2 illustriert. Das Aufnahmewerkzeug ist als zylinderförmiger Stößel oder quaderförmiger Schieber gebildet, der im Hohlkanal 31 der Kapillare 11 (Teilbild G) oder in einer offenen, rinnenförmigen Ausnehmung 34 des Sondenkörpers 11 (Teilbild H) verschiebbar angeordnet ist. Insbesondere die in den Teilbildern F bis H gezeigten Ausführungsformen der erfindungsgemäßen Sonde 10 besitzen den Vorteil, dass auf der Sonde 10 ein Flüssigkeitstropfen 81 als Schutz für Zellmaterial auf der Sonde 10 gebildet werden kann.

Eine schalen- oder löffelförmige Gestaltung des freien Endes 12 der Sonde 10 ist in Teilbild I illustriert. Am Ende des Sondenkörpers 11 ist ein Aufnahmewerkzeug 15 in Form eines Teils einer Hohlkugel gebildet, auf deren Innenseite die Aufnahmefläche 16 für mindestens eine Zelle 21 vorgesehen ist. Am Boden des Aufnahmewerkzeugs 15 befindet sich eine Öffnung 17, durch die die Zelle 21 aufgenommen werden kann. Die Aufnahme erfolgt durch eine Bewegung der Sonde 10 relativ zum Träger 80, ggf. unterstützt durch die natürliche Zellbewegung der Zelle 21. Bei einer abgewandelten Gestaltung kann auf die Öffnungen 17 verzichtet werden und stattdessen eine Aufnahme von Zellmaterial in das Aufnahmewerkzeug 15 über dessen Oberseite vorgesehen sein.

Teilbild J der Figur 2 zeigt Abstandshalter 18, die bei jeder der erfindungsgemäßen Sonden am freien Ende 12 oder an anderen Positionen des Sondenkörpers 11 vorgesehen sein können, um die Positionierung auf dem Träger 80 zu erleichtern. Ein besonderer Vorteil besteht darin, dass die erfindungsgemäße Verdrängungsbewegung der Sonde 10 durch Zellmaterial auch mit den Abstandshaltern 18 durchgeführt werden kann, ohne dass Zellen in unerwünschter Weise verletzt werden.

Der Durchmesser des Aufnahmewerkzeugs 15 gemäß den Teilbilder I und J beträgt bspw. 0.2 mm. Der Durchmesser der Öffnung 17 kann bspw. 1 µm bis 0.5 mm betragen.

Die Teilbilder K und L illustrieren, dass die Erfindung nicht nur zur Aufnahme von Einzelzellen, sondern auch zum Transfer von Zellmaterial, wie z. B. Zellmonolagen oder Zellkulturen 20 verwendet werden kann. Gemäß Teilbild K ist bspw. ein schaufelförmiges Aufnahmewerkzeug 15 vorgesehen, das analog zu Teilbild D geformt und bspw. am Ende eines rohrförmigen oder kastenförmigen Sondenkörpers vorgesehen ist. Die Breite der Aufnahmefläche 16 beträgt bspw. 10 µm bis 50 mm. Die seitlichen Ränder 17 bewirken eine Abgrenzung des vom Träger 80 aufgenommenen Zellmaterials von seiner Umgebung. Teilbild L zeigt das Einfahren der Sonde 10 in ein Mehrlagen-Zellsystem oder Gewebeverband, um aus diesem eine Schichtung von Zellen (Zelllage 24) zerstörungsfrei und ohne eine biochemische Behandlung abzuheben. Der Vortrieb (siehe Pfeil) des Werkzeugs erfolgt mit der genannten physiologischen Bezugsgeschwindigkeit der Zellen.

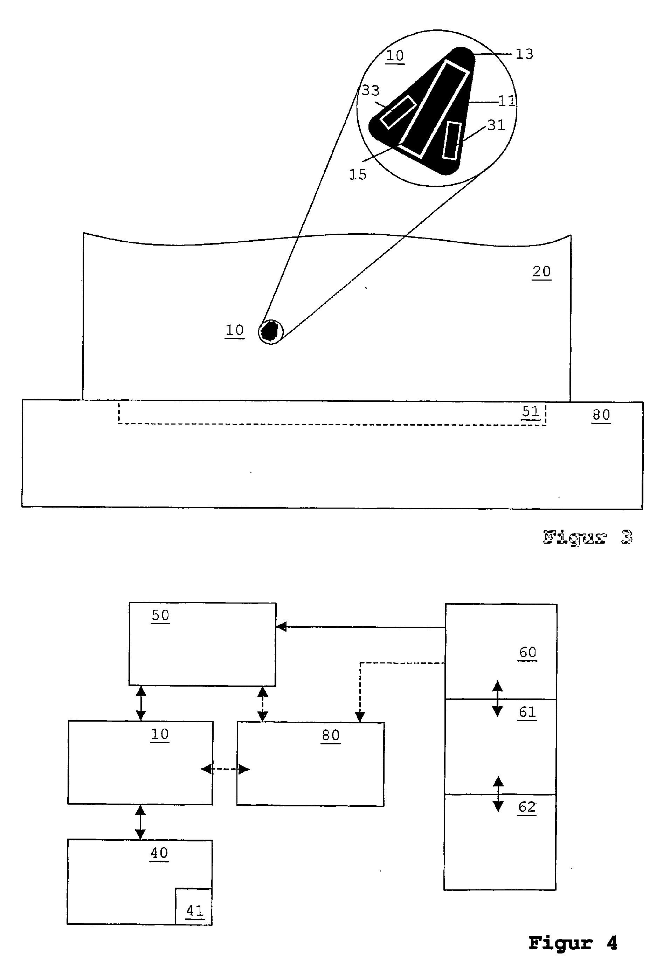

Figur 3 zeigt eine abgewandelte Ausführungsform der Erfindung, bei der die Sonde 10 (oben vergrößert dargestellt) ohne eine mechanische Verbindung nach außen vollständig vom Zellmaterial 20 umgeben wird. Das Zellmaterial 20 ist auf dem Träger 80 positioniert, in dem als Antriebseinrichtung eine Magneteinrichtung 51 integriert ist. Die Sonde 10 besitzt einen kegelförmigen Sondenkörper 11 mit Abrundungen 13 an den aneinander grenzenden Oberflächen. Die Abrundungen bilden in diesem Fall mehrere Vorderseiten. In den Sondenkörper 11 ist als Kraftelement ein Permanentmagnet 15 integriert. Durch Ausbildung geeignet geformter Magnetfelder mit der Magneteinrichtung 51, die bspw. eine Spulenanordnung zur Magnetfelderzeugung enthält, kann die Sonde 10 durch das Zellmaterial 20 gerichtet bewegt werden. Des Weiteren kann ein Driften der Sonde 10 unter der Wirkung interzellulärer Kräfte vorgesehen sein.

Das Bezugszeichen 31 bezieht sich auf einen Hohlraum im Sondenkörper 11, der bspw. mit einem Wirkstoff gefüllt ist. Durch eine Öffnung oder eine durchlässige Wand des Sondenkörpers 11 kann der Wirkstoff an den gewünschten Positionen im Zellmaterial 20 austreten. Das Bezugszeichen 33 bezieht sich auf einen Sensor, der in die Sonde 10 integriert ist. Der Sensor 33 umfasst bspw. einen pH-Sensor, Glukosesensor oder einen anderen Sensor für biologisch relevante Eigenschaften (Biosensor).

Erfindungsgemäß kann vorgesehen sein, dass die Sonde 10 gemäß Figur 3 zeitweilig mit einem externen Reservoir in Verbindung gesetzt wird, um bspw. den Hohlraum 31 mit einem Wirkstoff nachzufüllen. Hierzu wird entsprechend den oben erläuterten Prinzipien eine kapillarförmige Sonde durch das Zellmaterial 20 bis zur Sonde 10 bewegt und an diese angekoppelt. Analog kann in der Sonde 10 als Funktionseinheit ein elektrischer Stimulator mit einem elektrischen Energiereservoir angeordnet sein, das bei Bedarf über elektrische Leitungen mit einer externen Spannungsquelle verbunden wird. Diese Verbindung erfolgt durch Einführung elektrischer Leitungen entsprechend der erfindungsgemäßen Verdrängungsbewegung.

Ein erfindungsgemäßer Zellmanipulator umfasst gemäß der Blockdarstellung in Figur 4 mindestens eine Sonde 10 und mindestens eine Antriebseinrichtung 50 zur Ausübung einer Vortriebskraft an der Sonde 10. Die Antriebseinrichtung 50 enthält z. B. Piezokristalle, mit denen die Sonde 10 analog zu an sich bekannten Mikromanipulatorsystemen in verschiedenen Raumrichtungen mit einer Genauigkeit von < 100 nm positionierbar und bewegbar sind. Der Arbeitsweg der Sonde 10 kann zwischen 0,1 mm und einigen Zentimetern liegen.

Die Antriebseinrichtung 50 ist mit einer Positioniereinrichtung 60 verbunden. Die Positioniereinrichtung 60 ist ein mechanisch stabil und Mikrometer-genau relativ zu dem Zielzellsystem (Zellmaterial) fixierbares Bauteil, das es gestattet, die erforderlich langsame Bewegung der Sonde in das Zielzellsystem hinein oder heraus zu bewerkstelligen. Seine Aufgabe ist die stabile Lage des gesamten Injektionssystems gegenüber dem Zielzellsystem über die Dauer der Manipulation (Stunden, Tage oder auch Wochen) zu garantieren. Dies kann über eine justierbare Dreipunktlagerung auf dem Kultursystem (siehe Figur 5) oder eine Verankerung z.B. am Knochen des Schädels bei Zellinjektion ins Gehirn erreicht werden. Alternativ kann, wenn die Antriebseinrichtung 50 zur Bewegung des Trägers 80 des Zellmaterials eingerichtet ist, auch die Positioniereinrichtung 60 mit dem Trägers 80 verbunden sein (siehe gestrichelte Pfeile). Der Träger 80 ist bspw. eine Kulturschale (siehe Figur 5) oder ein Substrat in einem Kultivierungssystem.

Die Positioniereinrichtung 60 ist des Weiteren mit einer Steuereinrichtung 61 und einer Mess- und Anzeigeeinrichtung 62 verbunden. Die Steuereinrichtung 61 dient der Steuerung des Gesamtsystems und enthält einen Prozessor oder Computer. Die aktuellen und geplanten Positionen werden über Sensoren (Dehnungsmessstreifen an Aktoren, 4-Quadrantendetektion mittels Laserstrahl wie beim der Atomkraftmikroskopie oder in analoger Weise) erfolgen. Die Information wird Software-basiert verarbeitet und so dargestellt, dass die reale und geplante Bewegung auf einem Monitor zusammen mit zweckdienlichen Parametern dargestellt wird. Hierzu kann ein Kamerasystem mit mikroskopischer Vergrößerung und einer Zoom-Funktion vorgesehen sein.

Die Sonde 10 ist vorzugsweise mit einem Probenreservoir 40 verbunden, das ein Transportsystem 41 zur Bewegung einer Probe vom Reservoir 40 in die Sonde 10 enthält. Die in das Zellmaterial zu injizierende Probe ist bspw. eine Zellsuspension. Das Transportsystem 41 ist eine an sich bekannte Fördereinrichtungen wie z. B. eine Präzisions-Spritzenpumpe. Das Probenreservoir 40 für die Aufnahme der zu injizierenden Zellen kann eine Hamiltonspritze oder ein über ein totvolumenarmes 3-Wegesystem angeschlossenes Behältnis sein. Das Transportsystem 41 drückt beispielsweise über mechanische Kompression eine Zellsuspension in die Injektionswerkzeuge (Sonde 10). Dabei sind sehr geringe Volumina zu bewegen (Geschwindigkeit wenige µm/min). Es könne auch Spülvorgänge mit Waschlösungen (Geschwindigkeit 1 bis zu einigen 100 µm/s) ausgeführt werden. Dies kann über programmierbare Spritzenpumpen erreicht werden.

In Figur 5 sind weitere Einzelheiten des Zellmanipulators am Beispiel eines in vitro Systems für die Biotechnologie (Tissue Engineering) gezeigt. Ziel ist es, in einen Zellgewebeverband 20 mittels einer Mikrokapillare (Sonde 10) eine oder mehrere Zellen 22 aus einem Zellreservoir 40 zu injizieren. Die Sonde 10 ist über ein Kanalsystem mit dem Zellreservoir 40 verbunden. Gleichzeitig ist die Sonde 10 an eine Hamiltonspritze (Transportsystem 41 für die Zellsuspension 22) angeschlossen, die über einen Prozessor oder eine Computersteuerung angesprochen wird (nicht dargestellt). Das gesamte System befindet sich auf einer Arbeitsplattform 63, die sich nicht relativ zum Gewebeverband 20 bewegen kann, da sie über die Positioniereinrichtung 60 fest mit der Kulturschale 80 verbunden ist.

Die Grundjustage der Arbeitsplattform 63 erfolgt mit der Positioniereinrichtung 60. Als Vortriebssystem oder Antriebseinrichtung 50 für die Sonde 10 dient ein Piezorohr, das sich in der angegebenen Weise ausdehnen oder verkürzen kann und dadurch die Sonde 10 in den Gewebeverband 20 einführt bzw. herauszieht. Dargestellt ist ein Zielzellgebiet 23 (gepunkteter Ring), in den gerade eine Zelle 22 eingefügt wird. Am Schaft der Kapillar-Sonde 10 befindet sich ein Einzelzelldetektorsystem 33, mit dem die Zahl der injizierten Zellen erfasst werden kann (hier als optisches System dargestellt).

Der Zellmanipulator entsprechend Figur 5 wird wie folgt betätigt. In einem ersten Schritt wird die Positioniereinrichtung mit dem Zielgewebe 20 in eine feste Verbindung gebracht. Dies erfolgt durch Fixieren der Positioniereinheit an der Kulturschale 80 oder an der Oberfläche eines Organismus bzw. einem geeigneten Teil des Knochengerüstes. In einem zweiten Schritt wird das Mikroinjektionswerkzeug 10 an der Arbeitsplattform 61 befestigt und grob vorjustiert, so dass es kurz vor dem Zielgewebe- oder -zellsystem 20 mit seiner Spitze lokalisiert ist. Im nächsten Schritt wird die Kapillare der Sonde 10 mit der Nährlösung, einer physiologischen Lösung oder einem anderen geeigneten Fluid befüllt und an das Zielgewebe bis zu dessen Berührung herangeführt. Danach erfolgt der programmierte Vortrieb des Mikroinjektionswerkzeuges entsprechend der Geschwindigkeit von zum Beispiel weniger als 1 µm/h bis zu einigen 100 µm/h, bis der Zielbereich 23 erreicht ist. Entweder zu diesem Zeitpunkt oder früher werden die zu injizierenden Zellen aus dem Reservoir in das Mikroinjektionswerkzeug eingespült und über den Detektor beim Passieren des eingestochenen Teils erfasst, gezählt und ggf. charakterisiert. Durch Bewegen des Injektionswerkzeuges in alle drei Raumrichtungen mit der o. g. geringen Geschwindigkeit kann das System in ein neues Zielgebiet geführt werden, so dass in einer vorher bestimmbaren Weise Zellen dreidimensional im Gewebe positioniert werden. Nach erfolgter Injektion wird das Mikroinjektionswerkzeug wieder aus dem Zielgewebe entfernt. Die geringe Geschwindigkeit, die auch als "physiologische Geschwindigkeit" bezeichnet wird, erlaubt den Zellen des Gewebes an der Spitze des Injektionswerkzeuges das Lösen der Zelladhäsionskontakte, so dass es zu einer geordneten Verdrängung der Zellen kommt, nicht aber deren Verletzung.

Figur 6 zeigt ein Beispiel einer radialen Aufweitungsbewegung einer kapillarförmigen Sonde 10, deren Sondenkörper 11 aus einem elastischen Material (zum Beispiel Plastik, Gummi) oder einem nicht-elastisch aufweitbaren Material (zum Beispiel relativ zueinander verschiebbare Lamellen aus Stahl oder Kunststoff o. dgl.) besteht. Durch eine Druckerhöhung im Hohlkanal 31 der Sonde 10 kann deren Durchmesser erweitert werden, wobei eine verletzungsfreie Verdrängung der Zellen erfolgt. Der Durchmesser des Sondenkörpers 11 vergrößert sich mit der oben beschriebenen Vortriebsgeschwindigkeit.

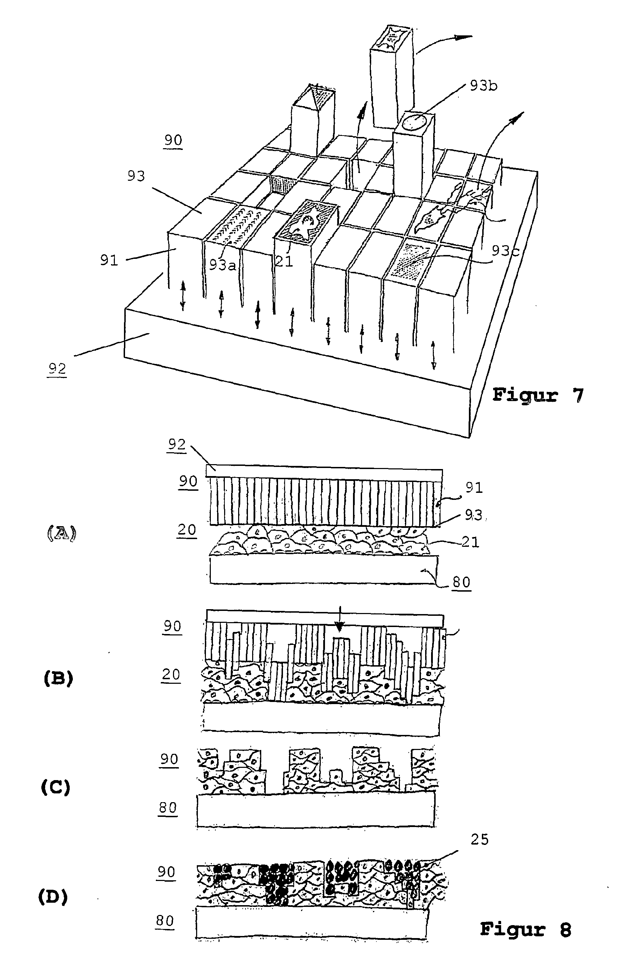

Figur 7 illustriert ein Ausführungsbeispiel einer Vielzahl erfindungsgemäß verwendeter Sonden 91, die ein Manipulationswerkzeug 90 bilden. Die Sonden sind Formelemente 91 des Manipulationswerkzeugs 90, die jeweils einzeln mit einer Antriebseinrichtung 92 verschiebbar sind, die an einem Basisteil (nicht dargestellt) angebracht ist. Jedes Formelement 91 besitzt eine langgestreckte Quaderform mit einer Oberseite 93. Die Gesamtzahl der Oberseiten 93 bilden die Oberfläche des Manipulationswerkzeugs 90, das zur Entnahme einzelner Zellen aus dem Zellmaterial 20 verwendet wird. Die Formelemente 91 bilden einen Werkzeugkörper. Die Oberseiten 93 besitzen typische Dimensionen im Bereich von 0.01 mm bis 5 mm. Sie sind abgeplattet ('unendlicher' Krümmungsradius) und haben ggf. abgerundete Ränder. Die Formelemente 91 sind so ausgerichtet, dass die Oberseiten 93 eine Matrixanordnung aus geraden Reihen und Spalten bilden. Die Verschiebung der Formelemente 91 relativ zum Basisteil erfolgt bspw. mit Stellmotoren oder piezoelektrischen Antrieben. Je nach dem gewählten Vorschub eines Formelements 91 wird die Werkzeugoberfläche strukturiert. Einzelne Formelemente 91 können von der Antriebseinrichtung 92 trennbar sein, um Zellen zu entnehmen.

Zur erfindungsgemäßen Entnahme von Zellen 21 aus einem Zellmaterial wird dieses zunächst auf der Werkzeugoberfläche, also der Gesamtheit der Oberseiten 93 angeordnet. Hierzu ist bspw. ein Aufwachsen aus einem Kultivierungsmedium in einem Kulturgefäß vorgesehen. Aus Übersichtlichkeitsgründen sind in Figur 7 nur einzelne Zellen 21 gezeigt. Anschließend erfolgt eine Beobachtung und Auswahl von Zellen, die aus dem Zellmaterial entnommen werden sollen. Die den ausgewählten Zellen entsprechenden Formelemente 91 werden vorgeschoben. Dieser Vorschub erfolgt mit der o. g. physiologischen Bezugsgeschwindigkeit, so dass eine verletzungsfreie Verdrängung und Trennung der ausgewählten Zelle erfolgt. Anschließend kann eine Ablösung des Formelements mit der Zelle vom Manipulationswerkzeug 90 erfolgen. Eine erfindungsgemäße Ablage einer Zelle 21 auf einem Zellmaterial kann analog mit der umgekehrten Schrittfolge vorgesehen sein.

Figur 7 illustriert auch, dass die einzelnen Oberseiten 93 der Formelemente 91 verschieden gebildet sein können, um an den jeweiligen Positionen das Zellmaterial zusätzlich zu modifizieren. Es können bspw. Mikrostrukturen (s. bei 93a) zur Verbesserung der Haftfähigkeit der Oberseite 93 oder zusätzliche Strukturelemente wie z. B. eine nach innen in das Formelement 91 gebildete Wölbung 93b zur verbesserten Trennung einer Zelle vom umgebenden Zellmaterial vorgesehen sein. Des Weiteren können einzelne oder alle Formelemente 91 eine adhäsionssteigernde Beschichtung 93c tragen.

Ein Manipulationswerkzeug 90 gemäß Figur 7 kann alternativ als Abdruckwerkzeug verwendet werden, wie dies in der Bildfolge gemäß Figur 8 illustriert ist. In einer Ausgangssituation gemäß Teilbild A befindet sich auf einem Träger 80 ein Zellmaterial 20, durch das entsprechend dem erfindungsgemäßen Verfahren ein Werkzeug geführt werden soll. Über der zunächst freien Oberfläche des Zellmaterials 20 wird das Manipulationswerkzeug 90 mit der Vielzahl von verschiebbaren Formelementen 91 angeordnet. Das Manipulationswerkzeug 90 wird an das Zellmaterial 20 angenähert, bis die in diesem Fall nach unten weisenden Oberseiten 93 das Zellmaterial 20 berühren. Anschließend erfolgt gemäß Teilbild B eine Verstellung der Oberfläche des Manipulationswerkzeugs 90 durch den gezielten Vorschub einzelner Formelemente 91. Die Vorschubbewegung erfolgt mit der o. g. physiologischen Bezugsgeschwindigkeit biologischer Zellen. Die einzelnen Formelemente 91 verdrängen die Zellen im Zellmaterial verletzungsfrei.

Anschließend wird gemäß Teilbild C das Manipulationswerkzeug 90 entfernt. Die Oberflächenform des Werkzeugs bleibt als komplementäre Struktur im Zellmaterial 20 bestehen. Für eine Erleichterung der Abtrennung des Manipulationswerkzeugs 90 vom Zellmaterial 20 können die Oberseiten 93 der Formelemente 91 mit einer Beschichtung versehen sein, auf der ein Anhaften von Zellen unterbunden wird. Die Beschichtung erfolgt bspw. mit dem Polymer Polyhema oder PTFE. Schließlich können die im Zellmaterial eingeprägten Lücken gemäß Teilbild D mit anderen Zellen oder einem synthetischen Matrixmaterial 25 befüllt werden.

Die Auswahl der Form und ggf. in das Zellmaterial zugeführten Zellen oder Zusatzstoffe 25 erfolgt je nach der konkreten Aufgabe im Rahmen des Tissue Engineering. Mit der Abfolge gemäß Figur 8 können bspw. Epithel-Zellen mit einer vorbestimmten Struktur mit Gewebe-Zellen in Verbindung gebracht werden.

Die in der vorstehenden Beschreibung, den Ansprüchen und den Zeichnungen offenbarten Merkmale der Erfindung können sowohl einzeln oder auch in Kombination für die Verwirklichung der Erfindung in ihren verschiedenen Ausgestaltungen von Bedeutung sein.

## Patentansprüche

1. Verfahren zur Bewegung einer Sonde (10, 91) durch ein Zellmaterial (20), das aus tierischen oder menschlichen Zellen (21) gebildet ist, ausgenommen menschliche embryonale Stammzellen, und das sich außerhalb eines tierischen oder menschlichen Organismus befindet, **dadurch gekennzeichnet, dass**
- die Sonde (10, 91) mit einer Vortriebsgeschwindigkeit im Bereich von 0.1 µm/h bis 1 mm/h bewegt wird, wobei
- die Sonde (10, 90) die Zellen (21) verletzungsfrei verdrängt.

2. Verfahren nach Anspruch 1, bei dem die Sonde (10, 91) mit einer Vortriebsgeschwindigkeit bewegt wird, die kleiner oder gleich einer molekularen Bindungsgeschwindigkeit der Zellen ist.

3. Verfahren nach Anspruch 1 oder 2, bei dem die Sonde (10, 91) einer permanent wirkenden Vortriebskraft ausgesetzt wird.

4. Verfahren nach Anspruch 3, bei dem die Vortriebskraft eine oder mehrere Kräfte umfasst, die aus der Gruppe von mechanischen Druckkräften, elektrischen Kräften, magnetischen Kräften und interzellulären Kräften ausgewählt sind.

5. Verfahren nach mindestens einem der vorgehenden Ansprüche, bei dem die Sonde (10, 91) durch Zellmaterial (20) bewegt wird, das ein Verbundmaterial umfasst, das aus den biologischen Zellen oder aus den biologischen Zellen und einem Matrixmaterial besteht.

6. Verfahren nach mindestens einem der vorgehenden Ansprüche, bei dem die Sonde (10, 91) eine langgestreckte Form mit einer Längsrichtung besitzt und in einer Richtung parallel zur Längsrichtung bewegt wird.

7. Verfahren nach mindestens einem der vorgehenden Ansprüche, bei dem mit der Sonde (10) eine Substanz in das Zellmaterial zugeführt wird.

8. Verfahren nach Anspruch 7, bei dem mit der Sonde (10) mindestens eine biologische Zelle (22) in das Zellmaterial zugeführt wird.

9. Verfahren nach Anspruch 8, bei dem die mindestens eine biologische Zelle (22) im gefrorenen Zustand in das Zellmaterial zugeführt wird.

10. Verfahren nach mindestens einem der vorgehenden Ansprüche, bei dem mit der Sonde (10, 91) mindestens eine Zelle aus dem Zellmaterial entnommen wird.

11. Verfahren nach mindestens einem der Ansprüche 8 bis 10, bei dem die mindestens eine Zelle bei der Zuführung in das Zellmaterial oder bei der Entnahme aus dem Zellmaterial eine natürliche Zellbewegung ausführt.

12. Verfahren nach mindestens einem der vorgehenden Ansprüche, bei dem mit der Sonde (10) Eigenschaften des Zellmaterials erfasst werden.

13. Verfahren nach mindestens einem der Ansprüche 1 bis 5, bei dem mit einer Vielzahl von Sonden (91), die jeweils ein Formelement eines Manipulationswerkzeugs (90) bilden, eine Formung des Zellmaterials (20) erfolgt.

14. Sonde (10, 91) zur Einführung in Zellmaterial (20) mit tierischen oder menschlichen Zellen (21), mit einem Sondenkörper (11), der durch das Zellmaterial beweglich ist,
**dadurch gekennzeichnet, dass**
- der Sondenkörper (11) mindestens an einer Vorderseite (13, 93), die bei Bewegung durch das Zellmaterial (20) in eine Bewegungsrichtung des Sondenkörpers (11) gerichtet ist, eine abgerundete Oberfläche besitzt und die abgerundete Oberfläche eine Beschichtung (14, 93c) trägt, die ein Anhaften von Zellen fördert, wobei
- die Sonde (10, 90) eingerichtet ist, bei Bewegung durch das Zellmaterial (20) die Zellen (21) verletzungsfrei zu verdrängen.

15. Sonde nach Anspruch 14, bei der die abgerundete Oberfläche einen Krümmungsradius besitzt, der größer als 10 µm ist.

16. Sonde nach mindestens einem der Ansprüche 14 oder 15, bei der der Sondenkörper (11) mit mindestens einem Funktionsteil (30) ausgestattet ist, das mindestens einen Hohlraum (31) im Sondenkörper (11) mit einer Öffnung (32), die in einer Oberfläche des Sondenkörpers gebildet ist, mindestens eine rinnenförmige Ausnehmung (34) und/oder mindestens einen Sensor (33) im Sondenkörper umfasst.

17. Sonde nach Anspruch 16, bei der der Sensor im Hohlraum (31) des Sondenkörpers (11) angeordnet ist.

18. Sonde nach mindestens einem der Ansprüche 14 bis 17, bei der der Sondenkörper (11) eine Kapillare mit einem ersten Ende (12), das die abgerundete Oberfläche aufweist, und einem zweiten Ende umfasst, das mit einer Reservoireinrichtung (40) verbunden ist.

19. Sonde nach Anspruch 18, bei der die Kapillare (11) an ihrem ersten Ende (12) ein Aufnahmewerkzeug (15) mit einer konkaven Aufnahmefläche (14) aufweist.

20. Sonde nach Anspruch 19, bei der in der Kapillare (11) ein Aufnahmewerkzeug (15) mit einer konvexen Aufnahmefläche (14) angeordnet ist.

21. Sonde nach Anspruch 16, bei der in der rinnenförmigen Ausnehmung (34) ein Aufnahmewerkzeug (15) angeordnet ist.

22. Sonde nach Anspruch 20 oder 21, bei der das Aufnahmewerkzeug (15) in der Kapillare (11) oder der rinnenförmigen Ausnehmung (34) beweglich angeordnet ist.

23. Sonde nach mindestens einem der Ansprüche 14 bis 17, bei der der Sondenkörper (11) einen Formkörper aufweist, dessen Oberfläche durch die abgerundete Oberfläche gebildet ist.

24. Sonde nach Anspruch 23, bei der der Sondenkörper (11) einen kugelförmigen Formkörper aufweist, der ein Aufnahmewerkzeug (15) bildet.

25. Sonde nach Anspruch 16, bei der das Funktionsteil (30) ein schalenförmiges Aufnahmewerkzeug (15) mit einer inneren Aufnahmefläche (14) umfasst.

26. Sonde nach Anspruch 25, bei der die Aufnahmefläche (14) eine Durchtrittsöffnung (17) aufweist.

27. Sonde nach mindestens einem der Ansprüche 14 bis 26, bei der am Ende (12) des Sondenkörpers (11) mindestens ein Abstandshalter (18) vorgesehen ist, mit dem der Sondenkörper (11) auf einem Träger (80) aufsetzbar ist.

28. Sonde nach mindestens einem der Ansprüche 14 bis 27, bei der der Sondenkörper (11) mindestens ein Kraftelement (15) aufweist, das zur Einwirkung einer äußeren Vortriebskraft eingerichtet ist.

29. Sonde nach Anspruch 28, bei der das Kraftelement (15) eine mechanische Halterung oder ein Magnetelement umfasst.

30. Zellmanipulator , der umfasst:
- mindestens eine Sonde (10, 91) nach mindestens einem der Ansprüche 14 bis 29, und
- mindestens eine Antriebseinrichtung (50, 92) zur Bewegung der mindestens einen Sonde (10).

31. Zellmanipulator nach Anspruch 30, der eine Positioniereinrichtung (60) zur Positionierung der Antriebseinrichtung (50, 92) und/oder eines Trägers (80) des Zellmaterials (20) aufweist.

32. Zellmanipulator nach Anspruch 30 oder 31, der eine Sensoreinrichtung zur Erfassung der Position der mindestens eine Sonde (10) und/oder von mindestens eine Zelle (21) des Zellmaterials (20) aufweist.

33. Zellmanipulator nach mindestens einem der Ansprüche 30 bis 32, bei dem der Träger (80) zur Aufnahme von Zellmaterial eingerichtet ist, wobei die Antriebseinrichtung dazu eingerichtet ist, die mindestens eine Sonde relativ zum Träger zu bewegen.

34. Zellmanipulator nach mindestens einem der Ansprüche 30 bis 33, bei dem die Antriebseinrichtung (50, 92) einen piezoelektrischen Antrieb oder einen Magnetantrieb aufweist.

35. Zellmanipulator nach mindestens einem der Ansprüche 30 bis 34, bei dem die Antriebseinrichtung (50) zur Bewegung der Sonde (10) mit Geschwindigkeiten im Bereich von 0.1 µm/h bis 1 mm/h eingerichtet ist.

36. Zellmanipulator nach mindestens einem der Ansprüche 30 bis 33, bei dem die Antriebseinrichtung (92) mit einer Vielzahl von einzeln verschiebbaren Sonden (91) ausgestattet ist.

37. Verwendung einer Hohlnadel mit einem abgerundeten, freien Ende, das eine Beschichtung (14) trägt, die ein Anhaften von Zellen fördert, zur verletzungsfreien Bewegung in Zellmaterial mit tierischen oder menschlichen Zellen, ausgenommen menschliche embryonale Stammzellen, wobei das Zellmaterial sich außerhalb einer tierischen oder menschlichen organismus befindet.

## Claims

1. A method for moving a probe (10, 91) through a cell material (20), which is formed from animal or human cells (21) excluding human embryonic stem cells and which is located outside an animal or human organism,
**characterized in that**
- the probe (10, 91) is moved at an advance velocity in the range from 0.1 µm/h to 1 mm/h, and
- the probe (10, 90) displaces the cells (21) without injury.

2. The method according to Claim 1, wherein the probe (10, 91) is moved at an advance velocity which is less than or equal to a molecular bonding velocity of the cells.

3. The method according to Claim 1 or 2, wherein the probe (10, 91) is subjected to a permanently acting advance force.

4. The method according to Claim 3, wherein the advance force comprises one or more forces which are selected from the group of mechanical pressure forces, electrical forces, magnetic forces, and intercellular forces.

5. The method according to at least one of the preceding claims, wherein the probe (10, 91) is moved through cell material (20), which comprises a composite material that is made of the biological cells or the biological cells and a matrix material.

6. The method according to at least one of the preceding claims, wherein the probe (10, 91) has an oblong shape having a longitudinal direction and is moved in a direction parallel to the longitudinal direction.

7. The method according to at least one of the preceding claims, wherein a substance is supplied into the cell material using the probe (10).

8. The method according to Claim 7, wherein at least one biological cell (22) is supplied into the cell material using the probe (10).

9. The method according to Claim 8, wherein the at least one biological cell (22) is supplied into the cell material in a frozen state.

10. The method according to at least one of the preceding claims, wherein at least one cell is removed from the cell material using the probe (10, 91).

11. The method according to at least one of Claim 8 through 10, wherein the at least one cell executes a natural cell movement as it is supplied into the cell material or as it is removed from the cell material.

12. The method according to at least one of the preceding claims, wherein properties of the cell material are detected using the probe (10).

13. The method according to at least one of Claims 1 to 5, wherein the cell material (20) is shaped using multiple probes (91), each of which forms a shaping element of a manipulation tool (90).

14. A probe (10, 91) for insertion into cell material (20) with animal or human cells (21), having a probe body (11), which is movable through the cell material,
**characterized in that**
- the probe body (11) has a rounded surface on at least one front side (13, 93), which points in a movement direction of the probe body (11) during movement through the cell material (20), wherein the rounded surface carrying a coating (14, 93c) that encourages adhesion of cells, wherein
- the probe (10, 91) is configured for displacing the cells (21) during the movement without injury.

15. The probe according to Claim 16, wherein the rounded surface has a radius of curvature that is larger than 10 µm.

16. The probe according to at least one of Claims 14 or 15, wherein the probe body (11) is equipped with at least one functional part (30), which comprises a cavity (31) in the probe body (11) having an opening (32), which is formed in a surface of the probe body, at least one groove-shaped recess (34), and/or at least one sensor (33) in the probe body.

17. The probe according to Claim 16, wherein the sensor is positioned in the cavity (31) of the probe body (11).

18. The probe according to at least one of Claims 14 to 17, wherein the probe body (11) comprises a capillary having a first end (12), which has the rounded surface, and a second end, which is connected to a reservoir device (40).

19. The probe according to Claim 18, wherein the capillary (11) has a receiving tool (15) having a concave receiving surface (16) on its first end (12).

20. The probe according to Claim 19, wherein a receiving tool (15) having a convex receiving surface (16) is positioned in the capillary (11).

21. The probe according to Claim 16, wherein a receiving tool (15) is positioned in the groove-shaped recess (34).

22. The probe according to Claim 20 or 21, wherein the receiving tool (15) is positioned so it is movable in the capillary (11) or in the groove-shaped recess (34).

23. The probe according to at least one of Claims 14 to 17, wherein the probe body (11) has a molded body whose surface is formed by the rounded surface.

24. The probe according to Claim 23, wherein the probe body (11) has a spherical molded body which forms a receiving tool (15).

25. The probe according to Claim 16, wherein the functional part (30) comprises a shell-shaped receiving tool (15) having an inner receiving surface (14).

26. The probe according to Claim 25, wherein the receiving surface (16) has a through opening (17).

27. The probe according to at least one of Claims 14 to 26, wherein at least one spacer (18) is provided at an end (12) of the probe body (11), using which the probe body (11) may be placed on a carrier (80).

28. The probe according to at least one of Claims 14 to 27, wherein the probe body (11) has at least one force element (15), which is adapted for the action of an external advance force.

29. The probe according to Claim 28, wherein the force element (15) comprises a mechanical holder or a magnetic element.

30. A cell manipulator, which comprises:
- at least one probe (10, 91) according to at least one of Claims 14 through 29, and
- at least one drive device (50, 92) for moving the at least one probe (10).

31. The cell manipulator according to Claim 30, which has a positioning device (60) for positioning the drive device (50, 92) and/or a carrier (80) of the cell material (20).

32. The cell manipulator according to Claim 30 or 31, which has a sensor device for detecting the position of the at least one probe (10) and/or at least one cell (21) of the cell material (20).

33. The cell manipulator according to at least one of Claims 30 to 32, wherein the carrier (80) is adapted for receiving cell material, the drive device being adapted for the purpose of moving the at least one probe in relation to the carrier.

34. The cell manipulator according to at least one of Claims 30 to 33, wherein the drive device (50, 92) has a piezoelectric drive or a magnetic drive.

35. The cell manipulator according to at least one of Claims 30 to 34, wherein the drive device (50) is adapted for moving the probe (10) at velocities in the range from 0.1 µm/h to 1 mm/h.

36. The cell manipulator according to at least one of Claims 30 to 33, wherein the drive device (92) is equipped with multiple individually displaceable probes (91).

37. A use of a hollow needle having a rounded, free end, which carries a coating which encourages adhesion of cells, for injury-free movement in cell material with animal or human cells, excluding human embryonic cells, wherein the cell material is located outside an animal or human organism.

## Revendications

1. Procédé de déplacement d'une sonde (10, 91) au travers d'une matière cellulaire (20) formée de cellules animales ou humaines (21), à l'exception de cellules souches embryonnaires humaines, et se trouvant à l'extérieur d'un organisme animal ou humain, **caractérisé**
- **en ce que** la sonde (10, 91) est mue avec une vitesse d'avance de l'ordre de 0,1 µm/h à 1 mm/h,
- la sonde (10, 90) refoulant les cellules (21) sans occasionner de lésion.

2. Procédé selon la revendication 1, où la sonde (10, 91) est mue avec une vitesse d'avance inférieure ou égale à une vitesse de liaison moléculaire des cellules.

3. Procédé selon la revendication 1 ou la revendication 2, où la sonde (10, 91) est soumise à une force d'avance exercée en permanence.

4. Procédé selon la revendication 3, où la force d'avance comprend une ou plusieurs forces sélectionnées dans le groupe des forces de pression mécaniques, des forces électriques, des forces magnétiques et des forces intercellulaires.

5. Procédé selon au moins une des revendications précédentes, où la sonde (10, 91) est mue au travers d'une matière cellulaire (20) comprenant une matière de liaison composée des cellules biologiques, ou des cellules biologiques et d'une matière matricielle.

6. Procédé selon au moins une des revendications précédentes, où la sonde (10, 91) présente une forme étirée en longueur avec une direction longitudinale, et est mue dans une direction parallèle à la direction longitudinale.

7. Procédé selon au moins une des revendications précédentes, où une substance est ajoutée par la sonde (10) à la matière cellulaire.

8. Procédé selon la revendication 7, où au moins une cellule biologique (22) est ajoutée par la sonde (10) à la matière cellulaire.

9. Procédé selon la revendication 8, où au moins une cellule biologique (22) en état de congélation est ajoutée à la matière cellulaire ;

10. Procédé selon au moins une des revendications précédentes, où au moins une cellule est prélevée de la matière cellulaire par la sonde (10, 91).

11. Procédé selon au moins une des revendications 8 à 10, où la ou les cellules exécutent un mouvement cellulaire naturel lors de l'ajout à la matière cellulaire ou du prélèvement de la matière cellulaire.

12. Procédé selon au moins une des revendications précédentes, où des propriétés de la matière cellulaire sont saisies par la sonde (10).

13. Procédé selon au moins une des revendications 1 à 5, où un formage de la matière cellulaire (20) est effectué avec une pluralité de sondes (91), qui forment chacune un élément de formage d'un outil de manipulation (90).

14. Sonde (10, 91) à introduire dans une matière cellulaire (20) avec des cellules animales ou humaines (21), comportant un corps de sonde (11) déplaçable au travers de la matière cellulaire, **caractérisée**
- **en ce que** le corps de sonde (11) présente une surface arrondie au moins sur une face avant (13, 93) orientée dans une direction de déplacement du corps de sonde (11) lors du déplacement au travers de la matière cellulaire (20), et en ce que la surface arrondie supporte un revêtement (14, 93c) favorisant une adhérence de cellules,
- la sonde (10, 90) étant agencée pour refouler les cellules (21) sans occasionner de lésion lors du déplacement au travers de la matière cellulaire (20).

15. Sonde selon la revendication 14, où la surface arrondie présente un rayon de courbure supérieur à 10 µm.

16. Sonde selon au moins une des revendications 14 et 15, où le corps de sonde (11) est pourvu d'au moins une pièce fonctionnelle (30) comprenant au moins une cavité (31) dans le corps de sonde (11), avec une ouverture (32) formée dans une surface du corps de sonde, au moins un évidement (34) en forme de gouttière et/ou au moins un capteur (33) dans le corps de sonde.

17. Sonde selon la revendication 16, où le capteur est disposé dans la cavité (31) du corps de sonde (11).

18. Sonde selon au moins une des revendications 14 à 17, où le corps de sonde (11) comprend un capillaire avec une première extrémité (12) comportant le surface arrondie, et avec une deuxième extrémité reliée à un dispositif de réservoir (40).

19. Sonde selon la revendication 18, où le capillaire (11) comporte un outil de réception (15) avec une surface de réception concave (14) à sa première extrémité (12).

20. Sonde selon la revendication 19, où un outil de réception (15) avec une surface de réception convexe (14) est disposé dans le capillaire (11).

21. Sonde selon la revendication 16, où un outil de réception (15) est disposé dans l'évidement en forme de gouttière (34).

22. Sonde selon la revendication 20 ou la revendication 21, où l'outil de réception (15) est disposé de manière à être mobile dans le capillaire (11) ou l'évidement en forme de gouttière (34).

23. Sonde selon au moins une des revendications 14 à 17, où le corps de sonde (11) comporte un corps de formage dont la surface est formée par la surface arrondie.

24. Sonde selon la revendication 23, où le corps de sonde (11) comporte un corps de formage sphérique formant un outil de réception (15).

25. Sonde selon la revendication 16, où la pièce fonctionnelle (30) comprend un outil de réception (15) en forme de coque avec une surface de réception (14) intérieure.

26. Sonde selon la revendication 25, où la surface de réception (14) comporte une ouverture de passage (17).

27. Sonde selon au moins une des revendications 14 à 26, où au moins un écarteur (18) est prévu à l'extrémité (12) du corps de sonde (11), avec lequel le corps de sonde (11) peut être déposé sur un support (80).

28. Sonde selon au moins une des revendications 14 à 27, où le corps de sonde (11) comporte au moins un élément de force (15) agencé pour exercer une force d'avance extérieure.

29. Sonde selon la revendication 28, où l'élément de force (15) comprend une fixation mécanique ou un élément magnétique.

30. Manipulateur cellulaire, comprenant :
- au moins une sonde (10, 91) selon au moins une des revendications 14 à 29, et
- au moins un dispositif d'entraînement (50, 92) pour le déplacement de la sonde ou des sondes (10).

31. Manipulateur cellulaire selon la revendication 30, comportant un dispositif de positionnement (60) pour le positionnement du dispositif d'entraînement (50, 92) et/ou d'un support (80) de la matière cellulaire (20).

32. Manipulateur cellulaire selon la revendication 30 ou la revendication 31, comportant un dispositif de capteur pour la saisie de position de la sonde ou des sondes (10) et/ou de la cellule ou des cellules (21) de la matière cellulaire (20).

33. Manipulateur cellulaire selon au moins une des revendications 30 à 32, où le support (80) est agencé pour la réception de matière cellulaire, le dispositif d'entraînement étant agencé pour déplacer la ou les sondes par rapport au support.

34. Manipulateur cellulaire selon au moins une des revendications 30 à 33, où le dispositif d'entraînement (50, 92) comporte un entraînement piézoélectrique ou un entraînement par aimant.

35. Manipulateur cellulaire selon au moins une des revendications 30 à 34, où le dispositif d'entraînement (50) est agencé pour déplacer la sonde (10) avec des vitesses de l'ordre de 0,1 µm/h à 1 mm/h.

36. Manipulateur cellulaire selon au moins une des revendications 30 à 33, où le dispositif d'entraînement (92) est pourvu d'une pluralité de sondes (91) individuellement déplaçables.

37. Utilisation d'une aiguille creuse, avec une extrémité libre arrondie supportant un revêtement (14) qui favorise une adhérence de cellules, pour un déplacement n'occasionnant pas de lésion dans de la matière cellulaire avec des cellules animales ou humaines, à l'exception de cellules souches embryonnaires humaines, la matière cellulaire se trouvant à l'extérieur d'un organisme animal ou humain.
